# EUROPÄISCHE PATENTSCHRIFT

(11) **EP 4 213 798 B1**
(45) Veröffentlichungstag und Bekanntmachung des Hinweises auf die Patenterteilung: **07.08.2024**
(21) Anmeldenummer: 21772717.1
(22) Anmeldetag: 30.08.2021
(51) Int. Cl.: A61K 8/22, A61K 8/44, A61K 8/64, A61Q 5/10

(54) **MITTEL ZUR OXIDATIVEN FARBVERÄNDERUNG VON KERATINISCHEN FASERN MIT GLYCYLGLYCIN UND AMINOSÄURE UND/ODER PROTEINHYDROLYSAT**
AGENTS FOR OXIDATIVELY CHANGING THE COLOR OF KERATIN FIBERS, CONTAINING GLYCYLGLYCINE AND AMINO ACID AND/OR PROTEIN HYDROLYSATE
AGENTS DE COLORATION OXYDATIVE DE FIBRES KÉRATINIQUES À L'AIDE DE GLYCYLGLYCINE ET D'AMINOACIDE ET/OU D'HYDROLYSAT PROTÉIQUE

(30) Priorität: 21.09.2020 DE 102020211752
(43) Veröffentlichungstag der Anmeldung: 26.07.2023
(73) Patentinhaber: Henkel AG & Co. KGaA, 40589 Düsseldorf (DE)
(72) Erfinder: GOUTSIS, Konstantin, 41363 Jüchen (DE); KESSLER-BECKER, Daniela, 51371 Leverkusen (DE)
(86) Internationale Anmeldenummer: PCT/EP2021/073861
(87) Internationale Veröffentlichungsnummer: WO 2022/058146

(56) Entgegenhaltungen:
- WO-A1-2007/068401
- FR-A1- 3 060 347
- US-A1- 2010 218 320
- US-A1- 2010 229 314

## Beschreibung

Die vorliegende Erfindung liegt im Bereich der Kosmetik und betrifft Mittel zur oxidativen Farbveränderung von keratinischen Fasern, insbesondere menschlichen Haaren, welche neben mindestens einem Oxidationsmittel die Kombination aus Glycylglycin und mindestens einer Aminosäure und/oder einem Proteinhydrolysat enthalten.

Die Veränderung von Form und Farbe der Haare stellt einen wichtigen Bereich der modernen Kosmetik dar. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Bei dunklem Ausgangshaar sind meist längere Anwendungszeiten und/oder wiederholte Blondiervorgänge notwendig, um das Haar über mehrere Nuancen aufzuhellen. Damit geht jedoch auch eine stärkere Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch.

Der Einsatz von Komplexbildnern bei oxidativen Farbveränderungen von keratinischen Fasern ist im Stand der Technik bekannt. Die Komplexbildner sollen unter anderem die Zersetzung des Wasserstoffperoxids durch in den Haarfasern angereicherte Metallionen verhindern.

So beschreibt die EP 1714634 A1 ein Haarbehandlungs-Kit zum Färben von menschlichen Haaren, umfassend ein erstes Kompartiment, welches einen Komplexbildner enthält, und ein zweites Kompartiment, welches Mittel zum Färben enthält. Durch den Einsatz eines Komplex-bildners sollen unerwünschte Reaktionen an und mit Haaren, die zu einer unerwünschten Erwärmung führen, verhindert werden.

In vielen auf dem Markt befindlichen Blondierprodukten werden als Komplexbildner HEDP (Etidronsäure) oder deren Salze oder aber EDTA (Ethylendiamin-Tetraacetat) oder dessen Salze eingesetzt. Sowohl HEDP als auch EDTA stabilieren Wasserstoffperoxid auf effektive Weise und komplexieren vorhandene Metallionen so effizient, dass eine bei der Anwendung stattfindende unerwünschte Temperaturerhöhung quasi vollständig vermieden wird. Der große Nachteil von HEDP und EDTA besteht jedoch in ihrer schlechten biologischen Abbaubarkeit. Gerade in jüngster Zeit achtet der Anwender zunehmend auf das ökologische Profil der von ihm verwendeten kosmetischen Produkte. So bevorzugt der Anwender vor allem möglichst nachhaltige Kosmetika mit biologisch abbaubaren Inhaltsstoffen.

Auch der Einsatz von Substanzen natürlicher Herkunft als Komplexbildner ist in der Literatur bereits bekannt. So beschreibt beispielsweise EP 1462090 A1 Mittel zum Blondieren, Färben bzw. Verformen von Haaren, welche die Kombination aus mindestens einem Oxidationsmittel und mindestens einer Polyhydroxycarbonsäure enthalten. Auch wenn die Polyhydroxycarbonsäuren der EP 1462090 A1 biologisch gut abbaubar sind, so sind die anwendungstechnischen Eigenschaften der in dieser Schriftt beschriebenen Mittel immer noch verbesserungswürdig, und insbesondere die Blondierleistung kann noch nicht als optimal betrachtet werden.

US 2010/218320 A1 offenbart eine nicht-oxidative Haarfärbezusammensetzung, umfassend mindestens ein Glycylglycinderivat und einen Direktfarbstoff.

US 2010/229314 A1 beschreibt Haarfärbemittel mit einem pH-Wert von 2 bis 12, die 0,1 bis 20 Gew.-% mindestens eines aromatischen Sulfon-Derivats und 0,01 bis 5 Gew-.% eines Glycylglycins enthalten.

In WO 2007/068401 A1 wird ein Haarbehandlungsmittel beschrieben, das einen Zucker, ein Oligopeptid und eine Aminosäure enthält, wobei es sich bei dem Oligopeptid auch um Glycylglycin handeln kann.

FR 3060347 A1 betrifft eine Zusammensetzung zum Aufhellen von Keratinfasern, die mindestens ein Oxidationsmittel (Wasserstoffperoxid) und mindestens ein organisches Amin enthält. Das organische Amin kann z.B. eine Aminosäure oder Glycylglycin sein.

Es war die Aufgabe der vorliegenden Erfindung, Haarbehandlungsmittel, insbesondere Blondiermittel bzw. Mittel zur oxidativen Farbveränderung, mit biologisch abbaubaren Komplexierungmitteln zu finden, die im Hinblick auf ihre Blondierleistung den aus dem Stand der Technik bekannten Blondier- bzw. Aufhellmitteln möglichst überlegen sind. Des Weiteren sollten die Blondiermittel eine ausreichend hohe Stabilität besitzen und sich auch bei der Anwendung auf Haaren mit höherem Metall- bzw. Schwermetallgehalt nicht zu stark erwärmen. Zudem sollte bei Anwendung der Komplexbildner in Aufhell- bzw. Blondiermitteln die Haarschädigung verringert werden.

Überraschenderweise hat sich nun herausgestellt, dass diese Aufgabe in vollem Umfang gelöst werden kann, wenn auf den Keratinfasern ein Mittel zur Anwendung kommt, welches neben dem für die Aufhellung verantwortlichen Oxidationsmittel (c) weiterhin die Kombination aus Glycylglycin (a) und mindestens einer Aminosäure und/oder einem Proteinhydrolysat (b) enthält.

Ein erster Gegenstand der vorliegenden Erfindung ist ein Mittel zur oxidativen Farbveränderung von keratinischen Fasern, insbesondere menschlichen Haaren, enthaltend
(a) Glycylglycin, und
(b) mindestens eine Aminosäure und/oder ein Proteinhydrolysat, und
(c) mindestens ein Oxidationsmittel.

### Keratinische Fasern

Unter keratinischen Fasern oder auch Keratinfasern sind dabei Pelze, Wolle, Federn und insbesondere menschliche Haare zu verstehen. Obwohl die Mittel in erster Linie zum Behandeln bzw. Aufhellen von Keratinfasern, insbesondere menschlichen Haaren, geeignet sind, steht prinzipiell einer Verwendung auch auf anderen Gebieten nichts entgegen.

### Mittel zur oxidativen Farbveränderung von keratinischen Fasern

Der erfindungsgemäß verwendete Begriff der "oxidativen Farbveränderung" umfasst prinzipiell jede mögliche Form der Behandlung, bei welcher die Färbung der Keratinfaser unter Anwendung mindestens eines Oxidationsmittels verändert wird. Insbesondere von dieser Erfindung umfasst sind Mittel zur oxidativen Färbung, zur aufhellenden Färbung, zur aufhellenden Nuancierung und zur Aufhellung, wobei die Aufhellung alternativ auch als Blondierung oder als Bleiche bezeichnet werden kann. Unter einer Aufhellung ist jedwede Form der Farbveränderung der Fasern zu verstehen, bei welcher die Keratinfasern im Vergleich zu der vor der Anwendung des Mittels vorhandenen Farbe eine hellere Färbung besitzen. Die Hellerfärbung der Haare wird durch mindestens ein im Mittel vorhandenen Oxidationsmittel bewirkt. Zusätzlich zu dem oder den Oxidationsmitteln können die erfindungsgemäßen Mittel zum Zwecke der Aufhellung auch noch farbgebende Komponenten, wie beispielsweise Oxidationsfarbstoffvorprodukte und/oder direktziehende Farbstoffe, enthalten. Durch die farbgebenden Komponenten kann der Farbausfall der resultierenden Färbung bei der Aufhellung leicht modifiziert werden. Bevorzugt sind diese farbgebenden Komponenten jedoch in so geringen Mengen im Mittel enthalten, dass der Farbeindruck der mit dem Mittel behandelten Keratinfasern dennoch heller als ihre Ursprungsfarbe ist. Entsprechende Färbetechniken können auch als färbende Blondierungen oder als nuancierende Blondierung bezeichnet werden.

Das Mittel enthält die erfindungswesentlichen Bestandteile (a), (b) und (c) bevorzugt in einem kosmetischen Träger. Als kosmetischer Träger kann für das Mittel beispielsweise ein geeigneter wässriger, alkoholischer oder wässrig-alkoholischer Träger eingesetzt werden. Zum Zwecke der Haarfärbung sind solche Träger beispielsweise Cremes, Emulsionen, Gele, Pasten oder auch tensidhaltige schäumende Lösungen, wie beispielsweise Shampoos, Schaumaerosole, Schaumformulierungen oder andere Zubereitungen, die für die Anwendung auf dem Haar geeignet sind.

Bevorzugt ist das erfindungsgemäße Mittel demzufolge ein Mittel zur oxidativen Farbveränderung von keratinischen Fasern, insbesondere menschlichen Haaren, enthaltend in einem kosmetischen Träger
(a) Glycylglycin, und
(b) mindestens eine Aminosäure und/oder ein Proteinhydrolysat, und
(c) mindestens ein Oxidationsmittel.

Bei diesem erfindungsgemäßen Mittel handelt es sich um ein anwendungsbereites Mittel, das in dieser Form zum Zweck der Blondierung bzw. Aufhellung oder oxidativen Färbung auf die Keratinfasern appliziert werden kann.

### Glycylglycin (a)

Als ersten erfindungswesentlichen Inhaltsstoff enthält das erfindungsgemäße Mittel Glycylglycin (a). Glycylglycin ist das Dipeptid der Aminosäure Glycin und besitzt die Struktur der Formel (I)

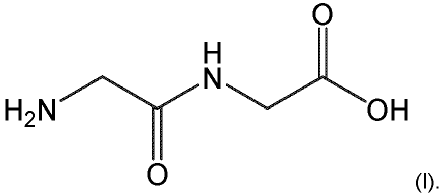

Glycylglycin besitzt die CAS-Nummer 556-50-3 und wird alternativ auch als Diglycin, GlyGly oder [(Aminoacetyl)amino]essigsäure bezeichnet.

Glycylglycin besizt eine Carbonsäuregruppe, die entweder in Form ihrer freien Säure oder aber auch in Form ihres physiologisch verträglichen Salzes vorliegen kann. Die physiologisch verträglichen Salze von Glycylglcin sind daher ebenfalls von dieser Erfindung umfasst.

Unter physiologisch verträglichen Salzen werden die Salze verstanden, die unter physiologischen Bedingungen ohne nachteilige Wirkung in Kosmetika eingesetzt werden können. Beispiele für ein physiologisch verträgliches Salz sind beispielsweise das Natriumsalz, das Kaliumsalz und das Ammoniumsalz.

Bei den zu dieser Erfindung führenden Arbeiten hat sich herausgestellt, dass die Blondierwirkung bei Wahl bestimmter Einsatzmengen von Glycylglycin (a) im erfindungsgemäßen Mittel noch weiter verbessert werden kann. Es ist daher ganz besonders bevorzugt, wenn das Mittel - bezogen auf das Gesamtgewicht des Mittels - 0,05 bis 10,0 Gew.-%, bevorzugt von 0,05 bis 7,5 Gew.-%, weiter bevorzugt von 0,05 bis 2,5 Gew.-% und ganz besonders bevorzugt von 0,05 bis 1,5 Gew.-% Glycylglycin (a) enthält.

Im Rahmen einer besonders bevorzugten Ausführungsform ist ein erfindungsgemäßes Mittel dadurch gekennzeichnet, dass es - bezogen auf das Gesamtgewicht des Mittels - 0,05 bis 10,0 Gew.-%, bevorzugt von 0,05 bis 7,5 Gew.-%, weiter bevorzugt von 0,05 bis 2,5 Gew.-% und ganz besonders bevorzugt von 0,05 bis 1,5 Gew.-% Glycylglycin (a) enthält.

### Aminosäure und/oder Proteinhydrolysat (b)

Als zweiten erfindungswesentlichen Bestandteil enthält das erfindungsgemäße Mittel mindestens eine Aminosäure und/oder ein Proteinhydrolysat (b).

Unter einer Aminosäure ist eine chemische Verbindung mit einer Aminogruppe und einer Carbonsäuregruppe zu verstehen. Zur Klasse der Aminosäuren zählen organische Verbindungen, die zumindest eine Aminogruppe (-NH₂ bzw. substituiert -NR₂) und eine Carboxygruppe (-COOH) als funktionelle Gruppen enthalten, also Strukturmerkmale der Amine und der Carbonsäuren aufweisen. Chemisch lassen sie sich nach der Stellung ihrer Aminogruppe zur Carboxygruppe unterscheiden - steht die Aminogruppe am C_{α}-Atom unmittelbar benachbart zur endständigen Carboxygruppe, nennt man dies α-ständig und spricht von α-Aminosäuren. Bevorzugt werden Carbonsäuren mit einer Gesamtzahl an C-Atomen von C2-C20, bevorzugter von C2-C15, besonders bevorzugt von C2-C10 eingesetzt.

Ganz besonder bevorzugte Aminosäuren sind ausgewählt aus Arginin, Glutaminsäure, Serin, Lysin, Histidin, Asparagin, Glutamin, Cystein, Methionin, Tryptophan, Serin, Alanin, Asparaginsäure, Glycin, Isoleucin, Leucin, Phenylalanin, Prolin, Threonin, Tyrosin und Valin sowie Mischungen dieser Aminosäuren.

Im Rahmen einer weiteren besonders bevorzugten Ausführungsform ist ein erfindungsgemäßes Mittel dadurch gekennzeichnet, dass es mindestens eine Aminosäure (b) aus der Gruppe aus Arginin, Glutaminsäure, Serin, Lysin, Histidin, Asparagin, Glutamin, Cystein, Methionin, Tryptophan, Serin, Alanin, Asparaginsäure, Glycin, Isoleucin, Leucin, Phenylalanin, Prolin, Threonin, Tyrosin und Valin enthält.

Bei Anwendung der erfindungsgemäßen Mittel konnnte im Vergleich zum Ausgangshaar dann eine besonders hohe Farberschiebung (d.h. ein besonder hoher delta-E-Wert) erzielt werden, wenn die erfindungsgemäßen Mittel mindestens eine Aminosäure (b) aus der Gruppe aus Arginin, Serin und Glutaminsäure enthielten.

Mittel, enthaltend mindestens eine Aminosäure (b) aus der Gruppe aus aus Arginin, Serin und Glutaminsäure sind im Hinblick auf die Verbesserung der Aufhelleistung daher ganz besonders bevorzugt.

Chirale Aminosäuren besitzen ein sterogenes Zentrum und können in spiegelbildlichen Formen auftreten. Beipsielsweise tritt Arginin in Form des L-Arginins und des D-Arginins auf. Sowohl die L-Form einer Aminosäure als auch ihre D-Form sowie die Gemische hiervon sind von der vorliegenden Erfindung umfasst. Im Rahmen der vorliegenden Erfindung können demnach beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Demzuvolge ist ein besonders bevorzugtes erfindungsgemäßes Mittel dadurch gekennzeichnet, dass es mindestens eine Aminosäure (b) aus der Gruppe aus L-Arginin, L-Glutaminsäure, L-Serin, L-Lysin, L-Histidin, L-Asparagin, L-Glutamin, L-Cystein, L-Methionin, L-Tryptophan, L-Serin, L-Alanin, L-Asparaginsäure, Glycin, L-Isoleucin, L-Leucin, L-Phenylalanin, L-Prolin, L-Threonin, L-Tyrosin und L-Valin enthält.

Für die Erzielung möglichst guter Ergebnisse, insbesondere guter Aufhell-Ergebnisse, werden die Aminosäure(n) (b) bevorzugt in bestimmten Mengenbereichen eingesetzt. Es hat sich als besonders vorteilhaft herausgestellt, wenn das Mittel - bezogen auf sein Gesamtgewicht - eine oder mehrere Aminosäuren (b) in einer Gesamtmenge von 0,1 bis 10,0 Gew.-%, bevorzugt 0,1 bis 5,0 Gew.-% enthält, weiter bevorzugt von 0,1 bis 2,5 Gew.-% und ganz besonders bevorzugt von 0,1 bis 1,0 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - eine oder mehrere Aminosäuren (b) in einer Gesamtmenge von 0,1 bis 10,0 Gew.-%, bevorzugt 0,1 bis 5,0 Gew.-% enthält, weiter bevorzugt von 0,1 bis 2,5 Gew.-% und ganz besonders bevorzugt von 0,1 bis 1,0 Gew.-% enthält.

Weiterhin gute Ergebnisse können erhalten werden, wenn im Mittel zusätzlich zu oder anstatt der Aminosäure mindestens ein Proteinhydrolysat eingesetzt wurde.

Proteinhydrolysate sind erfindungsgemäß Abbauprodukte von Proteinen, welche durch saure, basische oder enzymatische Reaktion hergestellt werden. Aufgrund des Herstellungsprozesses weisen Proteinhydrolysate eine Verteilung des Molekulargewichtes auf. Zu den erfindungsgemäßen Proteinhydrolysaten sind auch Oligopeptide zu zählen, da diese ebenfalls durch entsprechende Reaktionen aus Proteinen hergestellt werden können. Einzelne Aminosäuren, welche als diskrete Einzelverbindung vorliegen, zählen erfindungsgemäß nicht zu den Proteinhydrolysaten im Sinne dieser Erfindung. Erfindungsgemäß können Proteinhydrolysate sowohl pflanzlichen als auch tierischen oder marinen oder synthetischen Ursprungs eingesetzt werden.

Unter Proteinhydrolysaten im Sinne der vorliegenden Erfindung werden höchstens oligomere Verbindungen verstanden, die sich aus maximal 10 Aminosäuren zusammensetzen.

Da Proteinhydrolysate naturgemäß eine Mischung aus verschiedenen Aminosäuren und/oder Oligopeptiden umfassen, kann es abhängig von der Wahl des jeweiligen Proteinhydrolysats auch vorkommen, dass dieses Proteinhydrolysat auch Glycylglycin umfasst.

Im Unterschied zum Inhaltsstoff (a) beinhaltet das Proteinhydrolysat (b) das Glycylglycin jedoch nur als einen möglichen Bestandteil einer Mischung verschiedener Aminosäuren und Oligopeptide, wobei dieser Bestandteil im Vergleich zu den anderen Aminosäuren und Oligomeren in sehr geringer Menge vorhanden ist.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), ProSina^{®} (Croda) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Moringa-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda), Crotein^{®} (Croda) und Puricare^{®} LS 9658 von der Fa. Laboratoires Sérobiologiques erhältlich.

Ein weiteres ganz besonders gut geeignetes pflanzliches Proteinhydrolysat ist der Rostoff Gluadin^{®}Kera-P LM, der ein "Hydrolyzed Vegetable Protein" darstellt und beispielweise von der Firma BASF kommerziell erworben werden kann.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Weiterhin sind kationisierte Proteinhydrolysate zu den Proteinhydrolysaten zu zählen, wobei das zugrundeliegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Gute Ergebnisse konnten beobachtet werden, wenn des erfindungsgemäße Mittel mindestens ein Proteinhydrolysat enthielt, das ausgewählt ist aus der Gruppe der pflanzlichen Proteinhydrolysate, der Proteinhydrolysate des Elastins, des Kollagens, des Keratins, der Seide, des Milcheiweißes, Soja-, Mandel-, Erbsen-, Moringa-, Kartoffel- und Weizenproteinhydrolysaten.

Im Rahmen einer weiteren besonders bevorzugten Ausführungsform ist ein erfindungsgemäßes Mittel dadurch gekennzeichnet, dass es mindestens ein Proteinhydrolysat (b) enthält, das ausgewählt ist aus der Gruppe der pflanzlichen Proteinhydrolysate, der Proteinhydrolysate des Elastins, des Kollagens, des Keratins, der Seide, des Milcheiweißes, Soja-, Mandel-, Erbsen-, Moringa-, Kartoffel- und Weizenproteinhydrolysaten.

Für die Erzielung möglichst guter Ergebnisse, insbesondere guter Aufhell-Ergebnisse, werden die Proteinhydrolysate (b) bevorzugt in bestimmten Mengenbereichen eingesetzt. Es hat sich als besonders vorteilhaft herausgestellt, wenn das Mittel - bezogen auf das Gesamtgewicht des Mittels - ein oder mehrere Proteinhydrolysate (b) in einer Gesamtmenge von 0,1 bis 10,0 Gew.-%, bevorzugt 0,1 bis 5,0 Gew.-% enthält, weiter bevorzugt von 0,1 bis 2,5 Gew.-% und ganz besonders bevorzugt von 0,1 bis 1,0 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere Proteinhydrolysate (b) in einer Gesamtmenge von 0,1 bis 10,0 Gew.-%, bevorzugt 0,1 bis 5,0 Gew.-% enthält, weiter bevorzugt von 0,1 bis 2,5 Gew.-% und ganz besonders bevorzugt von 0,1 bis 1,0 Gew.-% enthält.

Ebenfalls erfindungsgemäße Proteinhydrolysate sind Oligopeptide. Oligopeptide können in den erfindungsgemäßen Haarbehandlungsmitteln aufgrund ihrer definierten Aminosäuresequenz bevorzugt sein.

Ein Oligopeptid, welches mindestens eine Aminosäuresequenz Glu-Glu-Glu 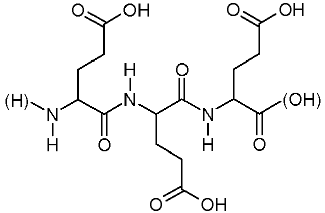 aufweist, wobei die Amino-Gruppe frei oder protoniert und die Carboxy-Gruppen frei oder deprotoniert vorliegen können, kann erfindungsgemäß besonders bevorzugt sein. In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie in der vorstehenden Formel, oder aber, daß die Aminosäuresequenz in einem Oligopeptid vorliegt, das noch weitere Anminosäuren umfaßt-je nachdem, wo die weitere(n) Aminosäure(n) gebunden ist/sind, sind die eingeklammerten Bestandteile der o.g. Formel durch den/die weiteren Aminosäurerest(e) ersetzt.

Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen. In erfindungsgemäß bevorzugten Haarbehandlungsmitteln umfaßt das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren. Je nachdem, ob weitere Aminosäuren an die Sequenz Glu-Glu-Glu gebunden sind und je nach Art dieser Aminosäuren kann die Molmasse des in den erfindungsgemäßen Mitteln enthaltenen Oligopeptids variieren. Erfindungsgemäß bevorzugte Haarbehandlungsmittel sind dadurch gekennzeichnet, daß das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist. Wie aus der bevorzugten Anzahl von Aminisäuren in den Oligopeptiden und dem bevorzugten Molmassenbereich zu ersehen ist, werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind. Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Haarbehandlungsmittel sind daher dadurch gekennzeichnet, daß das in ihnen enthaltene Oligopeptid mindestens eine Aminosäuresequenz Tyr-Glu-Glu-Glu 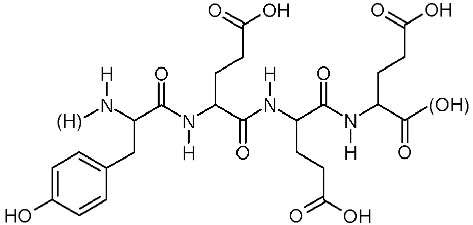 aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Haarbehandlungsmittel sind daher dadurch gekennzeichnet, daß das in ihnen enthaltene das Oligopeptid mindestens eine Aminosäuresequenz Glu-Glu-Glu-Ile 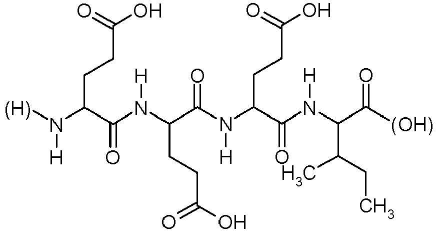 aufweist, wobei die Amino-Gruppe frei oder protoniert und die Carboxy-Gruppen frei oder deprotoniert vorliegen können.

Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in ihnen enthaltene Oligopeptid mindestens eine Aminosäuresequenz Tyr-Glu-Glu-Glu-Ile 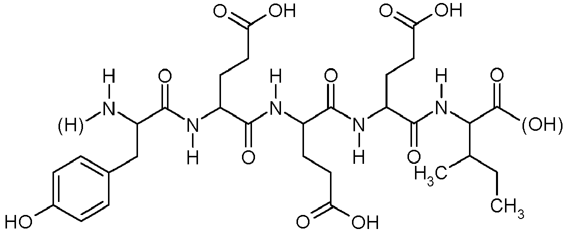 aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt

Noch weiter bevorzugte Oligopeptide enthalten zusätzlich Valin, das vorzugsweise an das Arginin gebunden vorliegt. Erfindungsgemäß weiter bevorzugte Haarbehandlungsmittel sind daher dadurch gekennzeichnet, daß das in ihnen enthaltene Oligopeptid mindestens eine Aminosäuresequenz Tyr-Glu-Glu-Glu-Ile-Arg-Val 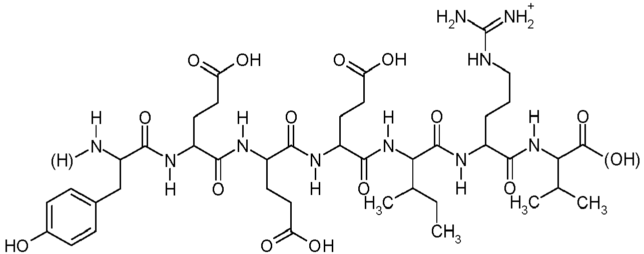 aufweist, wobei die Amino-Gruppen frei oder protoniert und die Carboxy-Gruppen frei oder deprotoniert vorliegen können. Noch weiter bevorzugte Oligopeptide enthalten zusätzlich Leucin, das vorzugsweise an das Valin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Tyr-Glu-Glu-Glu-Ile-Arg-Val-Leu 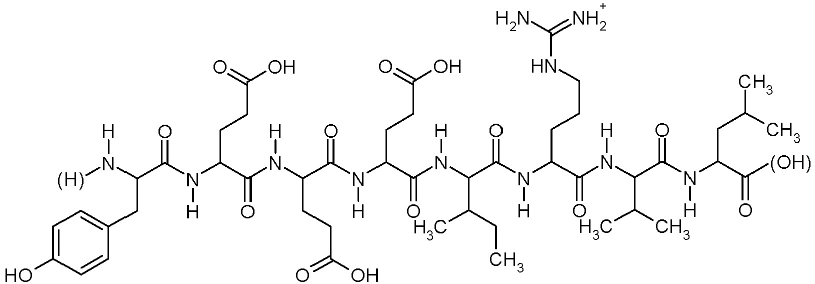 aufweist, wobei die Amino-Gruppen frei oder protoniert und die Carboxy-Gruppen frei oder deprotoniert vorliegen können.

Insbesondere bevorzugte Oligopeptide enthalten zusätzlich Leucin, das vorzugsweise an das Tyrosin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Leu-Tyr-Glu-Glu-Glu-Ile-Arg-Val-Leu 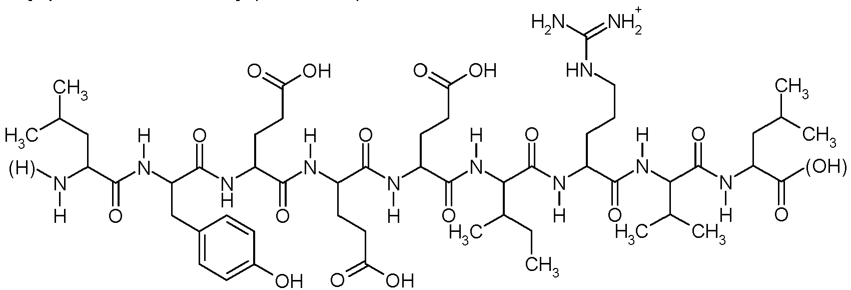 aufweist, wobei die Amino-Gruppen frei oder protoniert und die Carboxy-Gruppen frei oder deprotoniert vorliegen können.

### Gewichtsverhältnis (a)/(b)

Die zu dieser Erfindung führenden Arbeiten haben gezeigt, dass zwischen den Bestandteilen (a) und (b) eine synergistische Wirkung zu bestehen scheint. Bei Einsatz beider Bestandeile (a) und (b) in einem Mittel zur oxidativen Farbveränderung hat sich gezeigt, dass (a) und (b) zusammen die Aufhell-Leistung insbesondere dann ganz besonders stark verbessern, wenn sie in einem bestimmten Gewichtsverhältnis zueinander im Mittel vorhanden sind.

Aus diesem Grund hat es sich als ganz besonders bevorzugt herausgestellt, wenn das Gewichtsverhältnis aus allen im Mittel enthaltenen Komplexbildnern (a) der Formel (I) zu allen im Mittel enthaltenen Verbindungen aus der Gruppe der Aminosäuren und Proteinhydrolysate (b), d.h. das Gewichtsverhältnis (a)/(b), bei einem Wert von 0,1 bis 10, bevorzugt von 0,2 bis 5, weiter bevorzugt von 0,25 bis 4,0, und ganz besonders bevorzugt von 0,3 bis 1,0 liegt.

Im Rahmen weiteren bevorzugten Ausführungsform ist ein erfindungsgemäßes Mittel dadurch gekennzeichnet, dass das Gewichtsverhältnis aus dem im Mittel enthaltenen Glycylglycin (a) zu allen im Mittel enthaltenen Verbindungen aus der Gruppe der Aminosäuren und Proteinhydrolysate (b), d.h. das Gewichtsverhältnis (a)/(b), bei einem Wert von 0,1 bis 10, bevorzugt von 0,2 bis 5, weiter bevorzugt von 0,25 bis 4,0, und ganz besonders bevorzugt von 0,3 bis 1,0 liegt.

Im Rahmen weiteren ganz besonders bevorzugten Ausführungsform ist ein erfindungsgemäßes Mittel dadurch gekennzeichnet, dass das Gewichtsverhältnis aus dem im Mittel enthaltenen Glycylglycin (a) zu allen im Mittel enthaltenen Verbindungen aus der Gruppe der Aminosäuren (b), d.h. das Gewichtsverhältnis (a)/(b), bei einem Wert von 0,1 bis 10, bevorzugt von 0,2 bis 5, weiter bevorzugt von 0,25 bis 4,0, und ganz besonders bevorzugt von 0,3 bis 1,0 liegt.

### Mittel zum Aufhellen von Keratinfasern mit Oxidationsmittel (c)

Die Kombination der Inhaltsstoffe (a) und (b) zeigt ihr Potential in Mitteln zur oxidativen Farbveränderung von Keratinfasern, insbesondere in Mittel zur Aufhellung oder zur Blondierung von Haaren. Aus diesem Grund enthält das erfindungsgemäße Mittel als dritten erfindungswesentlichen Bestandteil mindestens ein Oxidationsmittel (c).

Unter einem Oxidationsmittel versteht der Fachmann eine Substanz oder eine Verbindung, die andere Stoffe oxidieren kann und dabei selbst reduziert wird. Oxidationsmittel können Elektronen aufnehmen, während Reduktionsmittel Elektronen abgeben. In der Haarbehandlung übliche Oxidationsmittel können ausgewählt werden aus der Gruppe der anorganischen und/oder organischen Peroxo-Verbindungen.

Zur Erzielung moderater Aufhelleffekte ist Wasserstoffperoxid das Oxidationsmittel der Wahl. Als Blondier- und Bleichmittel sind bevorzugte Mittel weiterhin dadurch gekennzeichnet, dass sie Wasserstoffperoxid und/oder eines seiner festen Anlagerungsprodukte an organische oder anorganische Verbindungen enthalten. Wird jedoch eine stärkere Aufhellung bzw. Blondierung gewünscht, wird Wasserstoffperoxid zusammen mit stärkeren Oxidationsmitteln wie beispielsweise Persulfaten (Natriumpersulfat, Kaliumpersulfat oder Ammoniumpersulfat) eingesetzt.

Im Rahmen einer besonders bevorzugten Ausführungsform ist ein erfindungsgemäßes Mittel dadurch gekennzeichnet, dass es
(c) mindestens ein Oxidationsmittel aus der Gruppe aus Wasserstoffperoxid, Ammoniumperoxodisulfat, Kaliumperoxodisulfat und Natriumperoxodisulfat enthält.

Rahmen dieser Ausführungsform besonders bevorzugt ist daher ein Mittel zur oxidativen Farbveränderung von keratinischen Fasern, insbesondere menschlichen Haaren, enthaltend in einem kosmetischen Träger
(a) Glycylglycin, und
(b) mindestens eine Aminosäure und/oder ein Proteinhydrolysat, und
(c) mindestens ein Oxidationsmittel aus der Gruppe aus Wasserstoffperoxid, Ammoniumperoxodisulfat, Kaliumperoxodisulfat und Natriumperoxodisulfat.

Unter Ammoniumperoxodisulfat, das alternativ auch als Ammoniumpersulfat bezeichnet werden kann, wird das Persufat mit der Summenfomel (NH₄)₂S₂O₈ verstanden.

Unter Kaliumperoxodisulfat, das alternativ auch als Kaliumpersulfat bezeichnet werden kann, wird das Persulfat mit der Summenformel K₂S₂O₈ verstanden.

Unter Natriumperoxodisulfat, das alternativ auch als Natriumpersulfat bezeichnet werden kann, wird das Persulfat mit der Summenformel Na₂S₂O₈ verstanden.

Im Rahmen einer besonders bevorzugten Ausführungsform ist ein erfindungsgemäßes Mittel dadurch gekennzeichnet, dass es enthält
(c1) Wasserstoffperoxid und
(c2) mindestens ein Persulfat aus der Gruppe aus Ammoniumperoxodisulfat, Kaliumperoxodisulfat und Natriumperoxodisulfat.

In einer weiteren bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 3 bis 12 Gew.-%ige Lösungen in Wasser verwendet. Erfindungsgemäß bevorzugte Mittel des ersten Erfindungsgegenstands sind dadurch gekennzeichnet, dass sie, bezogen auf das Gesamtgewicht des Mittels, (a) 0,1 bis 12,0 Gew.-% bevorzugt 0,5 bis 10,5 Gew.-%, weiter bevorzugt 1,0 bis 8,5 Gew.-%, noch weiter bevorzugt von 1,5 bis 7,0 Gew.-% und ganz besonders bevorzugt 1,5 bis 6,0 Gew.-% Wasserstoffperoxid enthalten.

Im Rahmen einer besonders bevorzugten Ausführungsform ist ein erfindungsgemäßes Mittel dadurch gekennzeichnet, es - bezogen auf das Gesamtgewicht des Mittels - (c) 0,1 bis 12,0 Gew.-% bevorzugt 0,5 bis 10,5 Gew.-%, weiter bevorzugt 1,0 bis 8,5 Gew.-%, noch weiter bevorzugt von 1,5 bis 7,0 Gew.-% und ganz besonders bevorzugt 1,5 bis 6,0 Gew.-% Wasserstoffperoxid enthält.

Auch das das oder die Persulfate werden bevorzugt in bestimmten Mengenbereichen im erfindungsgemäßen Mittel eingesetzt. Es hat sich als bevorzugt herausgestellt, wenn das Mittel - bezogen auf das Gesamtgewicht des Mittels - (c) ein oder mehrere Persulfate aus der Gruppe aus Ammoniumperoxodisulfat, Kaliumperoxodisulfat und Natriumperoxodisulfat in einer Gesamtmenge von 2,0 bis 40,0 Gew.-%, bevorzugt von 4,0 bis 30,0 Gew.-%, weiter bevorzugt von 6,0 bis 20,0 Gew.-% und ganz besonders von 8,0 bis 15,0 Gew.-% enthält.

Im Rahmen einer besonders bevorzugten Ausführungsform ist ein erfindungsgemäßes Mittel dadurch gekennzeichnet, dass es - bezogen auf das Gesamtgewicht des Mittels - (c) ein oder mehrere Persulfate aus der Gruppe aus Ammoniumperoxodisulfat, Kaliumperoxodisulfat und Natriumperoxodisulfat in einer Gesamtmenge von 2,0 bis 40,0 Gew.-%, bevorzugt von 4,0 bis 30,0 Gew.-%, weiter bevorzugt von 6,0 bis 20,0 Gew.-% und ganz besonders von 8,0 bis 15,0 Gew.-% enthält.

Im Rahmen dieser Ausführungsform besonders bevorzugt ist daher ein Mittel zur oxidativen Farbveränderung von keratinischen Fasern, insbesondere menschlichen Haaren, enthaltend in einem kosmetischen Träger
(a) Glycylglycin, und
(b) mindestens eine Aminosäure und/oder ein Proteinhydrolysat, und
(c1) 1,5 bis 6,0 Gew.-% Wasserstoffperoxid enthält, und
(c2) ein oder mehrere Persulfate aus der Gruppe aus Ammoniumperoxodisulfat,
Kaliumperoxodisulfat und Natriumperoxodisulfat in einer Gesamtmenge von 8,0 bis 15,0 Gew.-%,
wobei alle Mengenangaben in Gew.-% auf das Gesamtgewicht des Mittels bezogen sind.

Im Rahmen dieser Ausführungsform ganz besonders bevorzugt ist daher ein Mittel zur oxidativen Farbveränderung von keratinischen Fasern, insbesondere menschlichen Haaren, enthaltend in einem kosmetischen Träger
(a) Glycylglycin, und
(b) mindestens eine Aminosäure, und
(c1) 1,5 bis 6,0 Gew.-% Wasserstoffperoxid enthält, und
(c2) ein oder mehrere Persulfate aus der Gruppe aus Ammoniumperoxodisulfat, Kaliumperoxodisulfat und Natriumperoxodisulfat in einer Gesamtmenge von 8,0 bis 15,0 Gew.-%,
wobei alle Mengenangaben in Gew.-% auf das Gesamtgewicht des Mittels bezogen sind.

### Alkalisierungsmittel

Wie bereits zuvor beschrieben, handelt es sich bei dem erfindungsgemäßen Mittel des ersten Erfindungsgegenstands um ein anwendungsbereites Mittel zum Aufhellen bzw. Blondieren von Keratinfasern. Um eine ausreichenden Blondierwirkung zu erzielen, sind derartige Mittel üblicherweise alkalisch bis stark alkalisch eingestellt. Derart hohe pH-Werte sind erforderlich, um eine Öffnung der äußeren Schuppenschicht (Cuticula) zu gewährleisten und somit eine Penetration der aktiven Spezies (Wasserstoffperoxid und Persulfate) ins Haar zu ermöglichen.

Aus diesem Grund hat es sich als ganz besonders bevorzugt herausgestellt, wenn das erfindungsgemäße Mittel zusätzlich mindestens ein Alkalisieurngsmittel enthält.

Im Rahmen einer weiteren bevorzugten Ausführungsform ist ein erfindungsgemäßes Mittel dadurch gekennzeichnet, dass es mindestens ein Alkaliisierungsmittel enthält.

Bevorzugte Alkalisierungsmittel sind beispielsweise Ammoniak, Alkanolamine, basische Aminosäuren, sowie anorganische Alkalisierungsmittel wie (Erd-)Alkalimetallhydroxide, (Erd-)-Alkalimetallmetasilikate, (Erd-)Alkalimetallsilikate, (Erd-) Alkalimetallphosphate und (Erd-)-Alkali-metallhydrogenphosphate. Als Metallionen dienen bevorzugt Lithium, Natrium und/oder Kalium. Bevorzugte Alkalisierungsmittel sind (Erd-)Alkalimetallmetasilikate und (Erd-)Alkalimetallsilikate. Geeignete, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Alkanolamine als Alkalisierungsmittel werden bevorzugt ausgewählt aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Basische Aminosäuren als Alkalisierungsmittel werden bevorzugt ausgewählt aus der Gruppe, die gebildet wird aus L-Arginin, D-Arginin, D/L-Arginin, L-Lysin, D-Lysin, D/L-Lysin, L-Ornithin, D-Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel eingesetzt. Im Rahmen dieser Ausführungsform als Inhaltsstoff (b) eine basische Aminosäure eingesetzt werden, die sowohl - in Kombination mit dem Komplexbildner (a) - eine Verbesserung der Aufhellleistung bewirkt als auch für die Einstellung der besonders gut geeigneten alkalischen pH-Werte verantwortlich ist.

Besonders gut geeignete erfindungsgemäße Mittel besitzen einen pH-Wert im Bereich von 7,5 bis 11,5, bevorzugt von 8,0 bis 11,5, weiter bevorzugt von 8,5 bis 11,5 und ganz besonders bevorzugt von 9,0 bis 11,0. Bei den pH-Werten im Sinne der vorliegenden Erfindung handelt es sich um pH-Werte, die bei einer Temperatur von 22°C gemessen wurden. Die Messung des pH-Wertes kann beispielsweise mit einer kommerziell erhältlichen Glaselektrode gemessen werden.

Im Rahmen einer besonders bevorzugten Ausführungsform ist ein erfindungsgemäßes Mittel dadurch gekennzeichnet, dass es Wasser enthält und einen pH-Wert von 7,5 bis 11,5, bevorzugt von 8,0 bis 11,5, weiter bevorzugt von 8,5 bis 11,5 und ganz besonders bevorzugt von 9,0 bis 11,0 besitzt.

### Verzicht auf HEDP bzw. EDTA

Ziel der vorliegenden Anmeldung ist insbesondere der Verzicht auf die biologisch nicht abbaubaren Komplexbildner HEDP und EDTA. Aus diesem Grund enthalten die erfindungsgemäßen Mittel diese beiden Komplexbildner bevorzugt in besonders geringen Mengen. Ganz besonders bevorzugt sind die Mittel frei von diesen beiden Substanzen. Ganz besonders bevorzugt ist das Mittel auch im wesentlichen frei von den Salzen von HEDP und EDTA.

Im Rahmen einer weiteren bevorzugten Ausführungsform ist ein erfindungsgemäßes Mittel dadurch gekennzeichnet, dass - bezogen auf das Gesamtgewicht des Mittels - der Gesamtgehalt der im Mittel enthaltenen Etidronsäure und der Salze der Etidronäure unterhalb von 0,2 Gew.-%, bevorzugt unterhalb von 0,1 Gew.-%, weiter bevorzugt unterhalb von 0,05 Gew.-% und ganz besonders bevorzugt unterhalb von 0,001 Gew.-% liegt.

Im Rahmen einer weiteren bevorzugten Ausführungsform ist ein erfindungsgemäßes Mittel dadurch gekennzeichnet, dass - bezogen auf das Gesamtgewicht des Mittels - der Gesamtgehalt des im Mittel enthaltenen EDTA und der Salze des EDTA unterhalb von 0,2 Gew.-%, bevorzugt unterhalb von 0,1 Gew.-%, weiter bevorzugt unterhalb von 0,05 Gew.-% und ganz besonders bevorzugt unterhalb von 0,001 Gew.-% liegt

Etidronsäure wird alternativ auch als Hydroxyethan-1,1-diphosphonsäure bezeichnet und trägt die CAS-Nummer 2809-21-4.

EDTA wird alternativ auc als Ethylendiamin-tetraacetat bezeichnet und trägt die CAS-Nummern 6381-92-6 und 139-33-3 (wasserfrei).

### weitere Inhaltsstoffe

Zusätzlich zu den erfindungswesentlichen Inhaltsstoffen (a), (b) und (c) - sowie optional den Alkalisierungsmitteln - kann das erfindungsgemäße Mittel als nicht zwingende Bestandteile auch weitere Wirk- und Hilfsstoffe beinhalten. Diese werden nachfolgend beschrieben.

So können die Mittel ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; nichtionische Tensid, anionische Tenside, kationische Tenside, amphotere u/o zwitterionische Tenside, Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Oxidationsfarbstoffvorprodukte, direktziehende Farbstoffe; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren oxidativen Farbveränderung von Keratinfasern

Wie bereits zuvor beschrieben stellt das Mittel des ersten Erfindungsgegenstands ein Mittel zur oxidativen Farbveränderung von Keratinfasern dar. Ganz besonders bevorzugt handelt es sich bei bei dem Mittel um ein Aufhell- bzw. Blondiermittel dar. Dieses Aufhell-Blondiermittel stellt ein anwendungsbereites Mittel dar. Dementsprechend können die Mittel in einem Verfahren zur Behandlung, insbesondere zur Aufhellung der Keratinfasern eingesetzt werden.

Ein zweiter Gegenstand der vorliegenden Erfindung ist ein Verfahren zur zur oxidativen Farbveränderung von keratinischen Fasern, insbesondere menschlichen Haaren, wobei ein Mittel, wie es bei der Beschreibung des ersten Erfindungsgegenstands im Detail offenbart wurde, auf die Keratinfasern aufgetragen und nach einer Einwirkzeit wieder ausgespült wird.

Oxidationsmittel wie Wasserstoffoxid und Persulfate stellen hochreaktive Verbindungen da, die insbesondere im alkalischen Milieu nur eine begrenzte Stabilität besitzen. Aus diesem Grund wird das anwendungsbereite Blondiermittel üblicherweise kurz vor der Anwendung durch Vermischen von zwei oder mehreren separat verpackten Zubereitungen hergestellt.

Üblicherweise werden die Oxidationsmittel (a) und die Alkalisierungsmittel getrennt voneinander konfektioniert. Für den Komplexbildner (a) und die Aminosäure und/oder ein Proteinhydrolysate (b) sind nun verschiedene Arten der Konfektionierung denkbar.

So können Komplexbildner (a) und Aminosäure/Proteinhydrolysat (b) beispielsweise zusammen mit einem oder mehreren Persulfaten und getrennt vom Wasserstoffperoxid konfektioniert werden. Diese Ausführungsform ist insbesondere dann bevorzugt, wenn zur Herstellung des anwendungsbereiten Mittels nur zwei verschiedene Zubereitungen miteinander vermischt werden sollen.

In einer bevorzugten Ausführungsform ist ein Verfahren zur oxidativen Farbveränderung keratinischer Fasern dadurch gekennzeichnet, dass mindestens zwei getrennt voneinander verpackte Zubereitungen (A) und (B) zu einer Anwendungsmischung vermischt werden, diese auf die Fasern aufgetragen wird und nach einer Einwirkungszeit wieder abgespült wird, wobei
- die Zubereitung (A) Wasserstoffperoxid (c1) enthält, und
- die Zubereitung (B) enthält
   - Glycylglycin (a), und
   - mindestens ein Aminosäure und/oder ein Proteinhydrolysat (b), und
   - mindestens ein Persulfat (c2) aus der Gruppe aus Ammoniumperoxodisulfat, Kaliumperoxodisulfat und Natriumperoxodisulfat,
wobei die Inhaltstoffe (a) und (b) bereits bei der Beschreibung des ersten Erfindungsgegenstands im Detail offenbart wurden.

Die Zubereitungen (A) und (B) können entweder nur miteinander oder mit weiteren separat verpackten Zubereitungen unmittelbar vor der Anwendung zu einer Anwendungsmischung vermischt werden.

Wenn zur Herstellung des anwendungsbereiten Mittels drei verschiedene Zubereitungen miteinander vermischt werden sollen, kann es bevorzugt sein, das erste Oxidationsmittel Wasserstoffperoxid (c1) getrennt in einer ersten Zubereitung (A) bereit zu stellen, die Persulfate (c2) als zweites Oxidationsmittel getrennt in einer zweiten Zubereitung (B) bereitzustellen und weiterhin eine dritte Zubereitung (C) zur Verfügung zu stellen, welche Glycylglycin (a) und Aminosäure/Proteinhydrolysat (b) beinhaltet.

In einer weiteren bevorzugten Ausführungsform ist ein Verfahren zur oxidativen Farbveränderung keratinischer Fasern dadurch gekennzeichnet, dass mindestens drei getrennt voneinander verpackte Zubereitungen (A) und (B) und (C) zu einer Anwendungsmischung vermischt werden, diese auf die Fasern aufgetragen wird und nach einer Einwirkungszeit wieder abgespült wird, wobei
- die Zubereitung (A) Wasserstoffperoxid (c1) enthält, und
- die Zubereitung (B) mindestens ein Persulfat (c2) aus der Gruppe aus Ammoniumperoxodisulfat, Kaliumperoxodisulfat und Natriumperoxodisulfat enthält, und
- die Zubereitung (C) Glycylglycin (a) und mindestens eine Aminosäure und/oder ein Proteinhydrolysat (b) enthält,
wobei die Inhaltsstoffe (a) und (b) bereits bei der Beschreibung des ersten Erfindungsgegenstands im Detail offenbart wurden.

Die Zubereitungen (B), welche die Persulfate enthalten, sind bevorzugt pulverförmig. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) zu erleichtern.

Weiterin können die Zubereitungen (B) können die Persulfate auch in einem festen kosmetischen Träger enthalten. Ein fester kosmetischer Träger kann Salze der Kieselsäure, insbesondere Salze der Silicate und Metasilicate mit Ammonium, Alkalimetallen sowie Erdalkalimetallen enthalten. Insbesondere Metasilicate, die sich gemäß Formel (SiO₂)ₙ(M₂O)ₘ , wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO₃]²⁻ auffassen lassen, können bevorzugt eingesetzt werden. Natriummetasilicat der Formel [Na₂SiO₃]_{∞}, ist dabei besonders bevorzugt. Gleichfalls bevorzugt sind solche Silicate, die aus einem Silicat der Formel (SiO₂)ₙ(Na₂O)ₘ(K₂O)_{P} gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen Zubereitungen (B) so genannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage.

Schließlich können Zubereitungen (B) noch zusätzlich ein Entstaubungsmittel enthalten, die die Staubbildung der pulverförmigen Bestandteile verhindert. Hierzu können insbesondere inerte Öle eingesetzt werden. Bevorzugt enthalten die festen, kosmetischen Träger als Entstaubungsmittel Esteröle oder Mineralöle, bevorzugt Kohlenwasseröle, wie flüssiges Paraffinöl.

Die anwendungsbereiten Mittel werden unmittelbar vor der Anwendung auf dem Haardurch Mischen der zwei Zubereitungen (A) und (B) bzw. durch Vermischen der drei Zubereitungen (A) und (B) und (C) hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Das Mischen der zwei Zubereitungen (A) und (B) kann beispielsweise in einem Mengenverhältnis von 1:5 bis 5:1, bevorzugt 1:3 bis 3:1 und besonders 1:2 bis 2:1 erfolgen. Werden drei Zubereitungen (A), (B) und (C) miteinander vermischt, so sind ebenfalls verschiedene Mischungsverhältnisse möglich, die beispielsweise von 1:1:1 bis 3:1:1 bis 1:3:1 bis 1:1:3 bis 3:1:3 reichen können.

Der Begriff "unmittelbar" ist dabei als Zeitraum von wenigen Sekunden bis eine Stunde, vorzugsweise bis 30 min, insbesondere bis 15 min zu verstehen.

Die Zubereitungen (A), (B) sowie gegebenenfalls (C) werden in einem Verfahren zum Aufhellen von keratinischen Fasern, insbesondere menschlichen Haaren, angewendet, bei dem das Mittel auf die keratinhaltigen Fasern aufgebracht, für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch geringe Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist bevorzugt. Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark Tensid-haltigen Träger besitzt.

Die oben beschriebenen bevorzugten Ausführungsformen des Mittels gelten mutatis mutandis auch für das Verfahren.

### Beispiele

### 1. Herstellung des anwendungsbereiten Aufhellmittels durch Vermischen von drei Zubereitungen (A), (B) und (C)

Es wurden die folgenden Zubereitungen hergestellt (alle Angaben, sofern nichts anderes angegeben ist in Gew.-%).

| Zusammensetzung (A) | Gew.-% |
|---|---|
| Dinatriumpyrophosphat | 0,1 |
| Dipicolinsäure | 0,1 |
| Kaliumhydroxid (50 %ige wässrige Lösung) | 0,3 |
| Cetearylalkohol | 3,6 |
| Ceteareth-20 | 0,5 |
| Natriumlaurylsulfat | 0,3 |
| PEG-40 Castor Oil | 0,6 |
| Isopropylmyristat | 10,0 |
| Wasserstoffperoxid, 50 %ige wässrige Lösung | 23,2 |
| Wasser | ad 100 |

| Zubereitung (B) | Gew.-% |
|---|---|
| Kaliumpersulfat | 98,4 |
| Silica (fumed) | 1,4 |

| Zubereitung (C) | C1 Vergleich | C2 Erfindung | C3 Erfindung | C4 Erfindung |
|---|---|---|---|---|
| Cetearylalkohol | 5,75 | 5,75 | 5,75 | 5,75 |
| Lorol techn. (C12-C18-Fettalkohole) | 2,7 | 2,7 | 2,7 | 2,7 |
| Ceteareth-20 | 0,25 | 0,25 | 0,25 | 0,25 |
| Natriumlaurethsulfat (C12-14, 2EO 27% ige wässrige Lsg.) | 6 | 6 | 6 | 6 |
| Ammonium sulfat | 0,5 | 0,5 | 0,5 | 0,5 |
| Ammonia 25 % | 7,6 | 7,6 | 7,6 | 7,6 |
| Sodium silicate 40/42 | 0,5 | 0,5 | 0,5 | 0,5 |
| Kaliumhydroxid 50% | 1 | 1 | 1 | 1 |
| Glycylglycin | 0,2 | 0,2 | 0,2 | 0,2 |
| L-Serin | --- | 0,5 | --- | --- |
| L-Glutaminsäure | --- | --- | 0,5 | --- |
| L-Arginin | --- | --- | --- | 0,5 |
| Wasser | ad 100 | ad 100 | ad 100 | ad 100 |

### 2. Anwendung auf Haarsträhnen

Haarsträhnen (Kerling, Euronaturhaar 4-0) wurden farbmetrisch vermessen (Datacolor Spectraflash SF 450), vorshampooniert und getrocknet.

Zur Herstellung des anwendungsbereiten Aufhell- bzw. Blondiermittels wurden jeweils 60 g der Zubereitung (A) mit 20 g der Zubereitung (B) und 60 g der Zubereitung (C) vermischt. Die so erhaltene Anwendungsmischung wurde auf die Haarsträhnen appliziert, dort für 45 Minuten belassen und danach wieder mit Wasser ausgespült. Danach wurden die Haarsträhnen erneut farbmetrisch vermessen. Jede Anwendungsmischung wurde auf zwei Haarsträhnen appliziert.

Je höher der ΔL-Wert ist, desto größer ist die Aufhellung der Strähnen im Vergleich zum unbehandelten Haar.

Je höher der ΔE-Wert ist, desto größer ist die Farbverschiebung der Strähnen im Vergleich zum unbehandelten Haar.

| Anwendungsmischung | L | a | b | ΔL | ΔE |
|---|---|---|---|---|---|
| (A) + (B) + (C1) | 42,56 | 10,81 | 24,49 | 21,09 | 30,22 |
| 0,2% Glycylglycin | | | | | |
| (A) + (B) + (C1) | 42,42 | 10,77 | 24,24 | 20,95 | 29,95 |
| 0,2% Glycylglycin | | | | | |
| (A) + (B) + (C2) | 43,80 | 10,77 | 25,09 | 22,33 | 31,49 |
| 0,2% Glycylglycin + 0,5 % L-Serin | | | | | |
| (A) + (B) + (C2) | 43,27 | 10,99 | 25,09 | 21,80 | 31,17 |
| 0,2% Glycylglycin + 0,5 % L-Serin | | | | | |
| (A) + (B) + (C3) | 43,24 | 11,09 | 25,15 | 21,77 | 31,21 |
| 0,2% Glycylglycin + 0,5 % L-Glutaminsäure | | | | | |
| (A) + (B) + (C3) | 42,82 | 11,21 | 25,08 | 21,35 | 30,91 |
| 0,2% Glycylglycin + 0,5 % L-Glutaminsäure | | | | | |
| (A) + (B) + (C4) | 43,02 | 10,73 | 24,46 | 21,55 | 30,51 |
| 0,2% Glycylglycin + 0,5 % L-Arginin | | | | | |
| (A) + (B) + (C4) | 43,86 | 11,01 | 25,44 | 22,39 | 31,82 |
| 0,2% Glycylglycin + 0,5 % L-Arginin | | | | | |

Bei den Haarsträhnen, die unter Einsatz der Zubereitungen (C2), (C3) und (C4) blondiert wurden konnten besonders hohe ΔL-Werte und besonders hohe ΔE-Werte gemessen werden, d.h. mit diesen Formulierungen war die Farbverschiebung bzw. Farbveränderung im Vergleich zum Ausgangshaar am größten. Bei Einsatz der Zubereitungen (C2), (C3) und (C4) konnte somit die beste Aufhellleistung erzielt werden.

### 3. weitere Formulierungsbeispiele

Herstellung des anwendungsbereiten Aufhellmittels durch Vermischen von zwei Zubereitungen (A) und (B)

| Zusammensetzung (A) | Gew.-% |
|---|---|
| Dinatriumpyrophosphat | 0,1 |
| Dipicolinsäure | 0,1 |
| Kaliumhydroxid (50 %ige wässrige Lösung) | 0,3 |
| Cetearylalkohol | 3,6 |
| Ceteareth-20 | 0,5 |
| Natriumlaurylsulfat | 0,3 |
| PEG-40 Castor Oil | 0,6 |
| Isopropylmyristat | 10,0 |
| Wasserstoffperoxid, 50 %ige wässrige Lösung | 23,2 |
| Wasser | ad 100 |

| Zubereitung (B) (Gew-%) | B1 | B2 | B3 | B4 |
|---|---|---|---|---|
| Natriumsilicat | 33,0 | 33,0 | 33,0 | 33,0 |
| Magnesiumcarbonat | 12,8 | 12,8 | 12,8 | 12,8 |
| Natriumhexametaphosphat | 0,2 | 0,2 | 0,2 | 0,2 |
| Degalan RG S mv (Methyl methacrylate, Methacrylsäure Copolymer, Evonik) | 1,0 | 1,0 | 1,0 | 1,0 |
| Polyquaternium-4 | 0,3 | 0,3 | 0,3 | 0,3 |
| Silica (fumed) | 0,4 | 0,4 | 0,4 | 0,4 |
| Kaliumpersulfat | 32,0 | 32,0 | 32,0 | 32,0 |
| Ammoniumpersulfat | 10,0 | 10,0 | 10,0 | 10,0 |
| Ariabel Blue 300302 | 0,2 | 0,2 | 0,2 | 0,2 |
| Dimethicone, Dimethiconol | 1,5 | 1,5 | 1,5 | 1,5 |
| Glycylglycin | 0,5 | 1,0 | 2,0 | 3,0 |
| L-Lysin | 0,9 | --- | --- | --- |
| L-Arginin | --- | 1,5 | --- | --- |
| Glycin | --- | --- | 3,0 | --- |
| L-Valin | --- | --- | --- | 3,5 |
| Paraffinum Liquidum | ad 100 | ad 100 | ad 100 | ad 100 |

| Zubereitung (B) (Gew-%) | B5 | B6 | B7 | B8 |
|---|---|---|---|---|
| Natriumsilicat | 33,0 | 33,0 | 33,0 | 33,0 |
| Magnesiumcarbonat | 12,8 | 12,8 | 12,8 | 12,8 |
| Natriumhexametaphosphat | 0,2 | 0,2 | 0,2 | 0,2 |
| Degalan RG S mv (Methyl methacrylate, Methacrylsäure Copolymer, Evonik) | 1,0 | 1,0 | 1,0 | 1,0 |
| Polyquaternium-4 | 0,3 | 0,3 | 0,3 | 0,3 |
| Silica (fumed) | 0,4 | 0,4 | 0,4 | 0,4 |
| Kaliumpersulfat | 32,0 | 32,0 | 32,0 | 32,0 |
| Ammoniumpersulfat | 10,0 | 10,0 | 10,0 | 10,0 |
| Ariabel Blue 300302 | 0,2 | 0,2 | 0,2 | 0,2 |
| Dimethicone, Dimethiconol | 1,5 | 1,5 | 1,5 | 1,5 |
| Glygylglycin | 0,5 | 1,0 | 2,0 | 3,0 |
| L-Serin | 0,9 | --- | --- | --- |
| L-Valin | --- | 1,2 | --- | --- |
| L-Gluatminsäure | --- | --- | 3,1 | --- |
| L-Arginin | --- | --- | --- | 3,8 |
| Paraffinum Liquidum | ad 100 | ad 100 | ad 100 | ad 100 |

Durch Vermischen der Zubereitung (A) mit jeweils einer der Zubereitungen (B1) bis (B8) im Mischungsverhältnis 1:1 wurde ein anwendungsbereites Blondiermittel hergestellt.

### 4. Aufhellende Färbemittel, die durch Vermischen von zwei Zubereitungen (A) und (B) hergestellt werden

| Zubereitung (A) | Gew.-% | | | |
|---|---|---|---|---|
| Phosphorsäure 85 %ig | 0,04 | | | |
| Wasserstoffperoxid (50 %ige, wässrige Lösung) | 12,00 | | | |
| Emulgade F (INCI: Cetearylalcohol, PEG-40 Castor Oil, Sodium Cetearyl sulfate) | 2,10 | | | |
| Natriumbenzoat | 0,04 | | | |
| Dinatriumpyrophosphat | 0,30 | | | |
| Wasser | ad 100 | | | |

| Zubereitung (B) | B1 Gew.-% | B2 Gew.-% | B3 Gew.-% | B4 Gew.-% |
|---|---|---|---|---|
| Cetearyl Alcohol | 8,00 | 8,00 | 8,00 | 8,00 |
| Paraffinium Liquidum | 7,40 | 7,40 | 7,40 | 7,40 |
| Eumulgin B 3 (INCI: Ceteareth-30) | 1,30 | 1,30 | 1,30 | 1,30 |
| Acrylamidopropyltrimonium chloride /Acrylate Copolymer | 2,00 | 2,00 | 2,00 | 2,00 |
| p-Toluylendiamin Sulfat | 0,15 | 0,15 | 0,15 | 0,15 |
| Resorcin | 0,058 | 0,058 | 0,058 | 0,058 |
| m-Aminophenol | 0,016 | 0,016 | 0,016 | 0,016 |
| 3-Amino-2-methylamino-6-methoxypyridin | 0,005 | 0,005 | 0,005 | 0,005 |
| Kaliumhydroxid (50 %ig) | 0,7 | 0,7 | 0,7 | 0,7 |
| Natriumsilikat 42 (3,1 SiO₂ : Na₂O) | 0,5 | 0,5 | 0,5 | 0,5 |
| Ammoniak (25 Gew.-%ige wässrige Lösung) | 5,80 | 5,80 | 5,80 | 5,80 |
| Parfum | 0,40 | 0,40 | 0,40 | 0,40 |
| Glycylglycin | 0,4 | 0,6 | 0,8 | 1,5 |
| L-Arginine | 1,0 | 0,3 | --- | --- |
| L-Lysine | --- | 0,3 | 2,0 | 0,5 |
| L-Valin | --- | 0,6 | --- | 2,5 |
| Wasser | ad 100 | ad 100 | ad 100 | ad 100 |

Die Zubereitung (A) wurde jeweils im Mengenverhältnis 1:1 mit der Zubereitung (B) vermischt.

## Patentansprüche

1. Mittel zur oxidativen Farbveränderung von keratinischen Fasern, insbesondere menschlichen Haaren, enthaltend
(a) Glycylglycin, und
(b) mindestens eine Aminosäure und/oder ein Proteinhydrolysat, und
(c) mindestens ein Oxidationsmittel.

2. Mittel nach Anspruch 1, **dadurch gekennzeichnet, dass** es - bezogen auf das Gesamtgewicht des Mittels - 0,05 bis 10,0 Gew.-%, bevorzugt von 0,05 bis 7,5 Gew.-%, weiter bevorzugt von 0,05 bis 2,5 Gew.-% und ganz besonders bevorzugt von 0,05 bis 1,5 Gew.-% Glycylglycin (a) enthält.

3. Mittel nach einem der Ansprüche 1 bis 2, **dadurch gekennzeichnet, dass** es mindestens eine Aminosäure (b) aus der Gruppe aus Arginin, Glutaminsäure, Serin, Lysin, Histidin, Asparagin, Glutamin, Cystein, Methionin, Tryptophan, Serin, Alanin, Asparaginsäure, Glycin, Isoleucin, Leucin, Phenylalanin, Prolin, Threonin, Tyrosin und Valin enthält.

4. Mittel nach einem der Ansprüche 1 bis 3, **dadurch gekennzeichnet, dass** es - bezogen auf das Gesamtgewicht des Mittels - eine oder mehrere Aminosäuren (b) in einer Gesamtmenge von 0,1 bis 10,0 Gew.-%, bevorzugt 0,1 bis 5,0 Gew.-%, weiter bevorzugt von 0,1 bis 2,5 Gew.-% und ganz besonders bevorzugt von 0,1 bis 1,0 Gew.-% enthält.

5. Mittel nach einem der Ansprüche 1 bis 4, **dadurch gekennzeichnet, dass** es mindestens ein Proteinhydrolysat (b) enthält, das ausgewählt ist aus der Gruppe der pflanzlichen Proteinhydrolysate, der Proteinhydrolysate des Elastins, des Kollagens, des Keratins, der Seide, des Milcheiweißes, Soja-, Mandel-, Erbsen-, Moringa-, Kartoffel- und Weizenproteinhydrolysaten.

6. Mittel nach einem der Ansprüche 1 bis 5, **dadurch gekennzeichnet, dass** es - bezogen auf das Gesamtgewicht des Mittels - ein oder mehrere Proteinhydrolysate (b) in einer Gesamtmenge von 0,1 bis 10,0 Gew.-%, bevorzugt von 0,1 bis 5,0 Gew.-%, weiter bevorzugt von 0,1 bis 2,5 Gew.-% und ganz besonders bevorzugt von 0,1 bis 1,0 Gew.-% enthält.

7. Mittel nach einem der Ansprüche 1 bis 6, **dadurch gekennzeichnet, dass** das Gewichtsverhältnis aus dem im Mittel enthaltenen Glycylglycin (a) zu allen im Mittel enthaltenen Verbindungen aus der Gruppe der Aminosäuren und Proteinhydrolysate (b), d.h. das Gewichtsverhältnis (a)/(b), bei einem Wert von 0,1 bis 10, bevorzugt von 0,2 bis 5, weiter bevorzugt von 0,25 bis 4,0, und ganz besonders bevorzugt von 0,3 bis 1,0 liegt.

8. Mittel nach einem der Ansprüche 1 bis 7, **dadurch gekennzeichnet, dass** es (c) mindestens ein Oxidationsmittel aus der Gruppe aus Wasserstoffperoxid, Ammoniumperoxodisulfat, Kaliumperoxodisulfat und Natriumperoxodisulfat enthält.

9. Mittel nach einem der Ansprüche 1 bis 8, **dadurch gekennzeichnet, dass** es - bezogen auf das Gesamtgewicht des Mittels - (c) 0,1 bis 12,0 Gew.-%, bevorzugt 0,5 bis 10,5 Gew.-%, weiter bevorzugt 1,0 bis 8,5 Gew.-%, noch weiter bevorzugt von 1,5 bis 7,0 Gew.-% und ganz besonders bevorzugt 1,5 bis 6,0 Gew.-% Wasserstoffperoxid enthält.

10. Mittel nach einem der Ansprüche 1 bis 9, **dadurch gekennzeichnet, dass** es - bezogen auf das Gesamtgewicht des Mittels - (c) ein oder mehrere Persulfate aus der Gruppe aus Ammoniumperoxodisulfat, Kaliumperoxodisulfat und Natriumperoxodisulfat in einer Gesamtmenge von 2,0 bis 40,0 Gew.-%, bevorzugt von 4,0 bis 30,0 Gew.-%, weiter bevorzugt von 6,0 bis 20,0 Gew.-% und ganz besonders von 8,0 bis 15,0 Gew.-% enthält.

11. Mittel nach einem der Ansprüche 1 bis 10, **dadurch gekennzeichnet, dass** es Wasser enthält und einen pH-Wert von 7,5 bis 11,5, bevorzugt von 8,0 bis 11,5, weiter bevorzugt von 8,5 bis 11,5 und ganz besonders bevorzugt von 9,0 bis 11,0 besitzt.

12. Verfahren zur oxidativen Farbveränderung von keratinischen Fasern, insbesondere menschlichen Haaren, wobei ein Mittel nach einem der Ansprüche 1 bis 11 auf die Keratinfasern aufgetragen und nach einer Einwirkzeit wieder ausgespült wird.

## Claims

1. Agent for the oxidative color change of keratinous fibers, in particular human hair, containing
(a) glycylglycine, and
(b) at least one amino acid and/or a protein hydrolyzate, and
(c) at least one oxidizing agent.

2. The composition according to claim 1, **characterized in that** it contains - based on the total weight of the composition - from 0.05 to 10.0% by weight, preferably from 0.05 to 7.5% by weight, more preferably from 0.05 to 2.5% by weight and most preferably from 0.05 to 1.5% by weight of glycylglycine (a).

3. Agent according to one of claims 1 to 2, **characterized in that** it contains at least one amino acid (b) from the group consisting of arginine, glutaminacid, serine, lysine, Histidin, asparagine, glutamine, cysteine, methionine, tryptophan, serine, alanine, aspartic acid, glycine, isoleucine, leucine, phenylalanine, proline, threonine, tyrosine and valine.

4. Agent according to one of claims 1 to 3, **characterized in that** it contains - based on the total weight of the agent - one or more amino acids (b) in a total amount of 0.1 to 10.0% by weight, preferably 0.1 to 5.0% by weight, more preferably from 0.1 to 2.5% by weight and most preferably from 0.1 to 1.0% by weight.

5. Agent according to one of claims 1 to 4, **characterized in that** it contains at least one protein hydrolysate (b) selected from the group of vegetable protein hydrolysates, protein hydrolysates of elastin, collagen, keratin, silk, milk protein, soy, almond, pea, moringa, potato and wheat protein hydrolysates.

6. Agent according to one of claims 1 to 5, **characterized in that** it contains - based on the total weight of the agent - one or more protein hydrolysates (b) in a total amount of from 0.1 to 10.0% by weight, preferably from 0.1 to 5.0% by weight, more preferably from 0.1 to 2.5% by weight and most preferably from 0.1 to 1.0% by weight.

7. Agent according to one of claims 1 to 6, **characterized in that** the weight ratio of the glycylglycine (a) contained in the agent to all compounds from the group of amino acids and protein hydrolysates (b) contained in the agent, i.e. the weight ratio (a)/(b), is from 0.1 to 10, preferably from 0.2 to 5, more preferably from 0.25 to 4.0, and most preferably from 0.3 to 1.0.

8. Agent according to one of claims 1 to 7, **characterized in that** it
(c) contains at least one oxidizing agent from the group consisting of hydrogen peroxide, ammonium peroxodisulfate, potassium peroxodisulfate and sodium peroxodisulfate.

9. Agent according to one of claims 1 to 8, **characterized in that** it contains - based on the total weight of the agent - (c) 0.1 to 12.0% by weight, preferably 0.5 to 10.5% by weight, more preferably 1.0 to 8.5% by weight, still more preferably from 1.5 to 7.0% by weight and very particularly preferably 1.5 to 6.0% by weight of hydrogen peroxide.

10. Agent according to one of claims 1 to 9, **characterized in that** it contains - based on the total weight of the agent - (c) one or more persulfates from the group consisting of ammonium peroxodisulfate, potassium peroxodisulfate and sodium peroxodisulfate in a total amount of from 2.0 to 40.0% by weight, preferably from 4.0 to 30.0% by weight, more preferably from 6.0 to 20.0% by weight and very particularly from 8.0 to 15.0% by weight.

11. Agent according to one of claims 1 to 10, **characterized in that** it contains water and has a pH value of 7.5 to 11.5, preferably of 8.0 to 11.5, more preferably of 8.5 to 11.5 and most preferably of 9.0 to 11.0.

12. Process for the oxidative color change of keratinous fibers, in particular human hair, wherein an agent according to one of claims 1 to 11 is applied to the keratin fibers and rinsed out again after an exposure time.

## Revendications

1. Composition pour la modification oxydative de la couleur des fibres kératiniques, en particulier des cheveux humains, contenant
(a) la glycylglycine, et
(b) au moins un acide aminé et/ou un hydrolysat de protéine, et
(c) au moins un agent oxydant.

2. Agent selon la revendication 1, **caractérisé en ce qu'**il contient - par rapport au poids total de l'agent - de 0,05 à 10,0 % en poids, de préférence de 0,05 à 7,5 % en poids, plus préférentiellement de 0,05 à 2,5 % en poids et tout particulièrement de 0,05 à 1,5 % en poids de glycylglycine (a).

3. Agent selon l'une des revendications 1 à 2, **caractérisé en ce qu'**il contient au moins un acide aminé (b) choisi parmi l'arginine, l'acide glutamin; sérine, la lysine, l'histidin, l'asparagine, la glutamine, la cystéine, la méthionine, le tryptophane, la sérine, l'alanine, l'acide aspartique, la glycine, l'isoleucine, la leucine, la phénylalanine, la proline, la thréonine, la tyrosine et la valine.

4. Agent selon l'une des revendications 1 à 3, **caractérisé en ce qu'**il contient - par rapport au poids total de l'agent - un ou plusieurs acides aminés (b) en une quantité totale de 0,1 à 10,0 % en poids, de préférence de 0,1 à 5,0 % en poids, de préférence encore de 0,1 à 2,5 % en poids et de manière tout à fait préférée de 0,1 à 1,0 % en poids.

5. Agent selon l'une des revendications 1 à 4, **caractérisé en ce qu'**il contient au moins un hydrolysat de protéines (b) choisi dans le groupe des hydrolysats de protéines végétales, des hydrolysats de protéines d'élastine, de collagène, de kératine, de soie, de protéines de lait, des hydrolysats de protéines de soja, d'amande, de pois, de moringa, de pomme de terre et de blé.

6. Agent selon l'une des revendications 1 à 5, **caractérisé en ce qu'**il contient - par rapport au poids total de l'agent - un ou plusieurs hydrolysats de protéines (b) en une quantité totale de 0,1 à 10,0 % en poids, de préférence de 0,1 à 5,0 % en poids, de préférence encore de 0,1 à 2,5 % en poids et de manière tout à fait préférée de 0,1 à 1,0 % en poids.

7. Agent selon l'une des revendications 1 à 6, **caractérisé en ce que** le rapport pondéral entre la glycylglycine (a) contenue dans l'agent et tous les composés du groupe des acides aminés et des hydrolysats de protéines (b) contenus dans l'agent, c'est-à-dire le rapport pondéral (a)/(b), a une valeur de 0,1 à 10, de préférence de 0,2 à 5, plus préférablement de 0,25 à 4,0, et tout particulièrement de 0,3 à 1,0.

8. Agent selon l'une des revendications 1 à 7, **caractérisés en ce qu'**ils sont (c) contient au moins un agent oxydant choisi dans le groupe constitué par le peroxyde d'hydrogène, le peroxodisulfate d'ammonium, le peroxodisulfate de potassium et le peroxodisulfate de sodium.

9. Agent selon l'une des revendications 1 à 8, **caractérisé en ce qu'**il contient - par rapport au poids total de l'agent - (c) de 0,1 à 12,0 % en poids, de préférence de 0,5 à 10,5 % en poids, de préférence encore de 1,0 à 8,5 % en poids, de préférence encore de 1,5 à 7,0 % en poids et de manière tout à fait préférée de 1,5 à 6,0 % en poids de peroxyde d'hydrogène.

10. Agent selon l'une des revendications 1 à 9, **caractérisé en ce qu'**il contient - par rapport au poids total de l'agent - (c) un ou plusieurs persulfates choisis dans le groupe constitué par le peroxodisulfate d'ammonium, le peroxodisulfate de potassium et le peroxodisulfate de sodium en une quantité totale de 2,0 à 40,0 % en poids, de préférence de 4,0 à 30,0 % en poids, plus préférablement de 6,0 à 20,0 % en poids et tout particulièrement de 8,0 à 15,0 % en poids.

11. Agent selon l'une des revendications 1 à 10, **caractérisé en ce qu'**il contient de l'eau et possède un pH de 7,5 à 11,5, de préférence de 8,0 à 11,5, plus préférentiellement de 8,5 à 11,5 et tout particulièrement de 9,0 à 11,0.

12. Procédé de modification oxydative de la couleur de fibres kératiniques, en particulier de cheveux humains, dans lequel on applique sur les fibres kératiniques un produit selon l'une des revendications 1 à 11 et on le rince après un temps d'action.
